# EUROPÄISCHE PATENTSCHRIFT

(11) **EP 1 615 571 B1**
(45) Veröffentlichungstag und Bekanntmachung des Hinweises auf die Patenterteilung: **20.10.2010**
(21) Anmeldenummer: 04726226.6
(22) Anmeldetag: 07.04.2004
(51) Int. Cl.: A61B 17/74

(54) **MARKNAGEL ZUR FIXATION VON FEMUR-FRAKTUREN**
INTRAMEDULLARY NAIL FOR FEMUR FRACTURE FIXATION
CLOU INTRAMEDULLAIRE POUR FIXATION DE FRACTURES DU FEMUR

(30) Priorität: 09.04.2003 CH 6482003
(43) Veröffentlichungstag der Anmeldung: 18.01.2006
(73) Patentinhaber: Synthes GmbH, 4436 Oberdorf (CH)
(72) Erfinder: ASCHMANN, Felix, 4052 Basel (CH)
(74) Vertreter: Rosenich, Paul
(86) Internationale Anmeldenummer: PCT/IB2004/001064
(87) Internationale Veröffentlichungsnummer: WO 2004/089232

(56) Entgegenhaltungen:
- US-A- 5 454 813
- US-A- 6 077 264
- US-A1- 2002 045 900
- US-B1- 6 261 290

## Beschreibung

Die Erfindung betrifft einen Marknagel zur Fixation von Frakturen des proximalen Femur, mit einem vom proximalen Ende in den Markraum einführbaren Femurnagel und einer durch eine schräg zur Längsachse des Femurnagels verlaufende Durchgangsbohrung von lateral in den Femurnagel einführbaren Schenkelhalsschraube, wobei eine die axiale Bewegung der Schenkelhalsschraube in der Durchgangsbohrung des Femurnagels ermöglichende Drehsicherung vorgesehen ist, wobei die Schenkelhalsschraube wenigstens eine in axialer Richtung verlaufende Längsnut aufweist und ein Verriegelungselement mit wenigstens einem zur Achse der Schenkelhalsschraube etwa parallel gerichteten Schenkel vorgesehen ist, der von lateral in die Längsnuten der Schenkelhalsschraube einführbar ist.

Bei Frakturen des proximalen Femur muss das proximale Fragment (im wesentlichen der Femurkopf) gegenüber dem Femurschaft richtig orientiert und fixiert werden, also lage- und rotationsstabil gemacht werden.

Als Befestigungselemente kennt man Schrauben und Klingen, insbesondere sogenannte Spiralklingen. Schrauben geben sehr guten Halt in axialer Richtung, mässigen Halt in radialer Richtung (senkrecht zur Schraubenachse) und nahezu keinen Halt gegen Rotation um die Schraubenachse. Dem entgegengesetzt geben Klingen sehr guten Halt in radialer Richtung und auch gegen Rotation um die Klingenachse, aber nahezu keinen Halt in axialer Richtung.

Es sind beispielsweise aus der EP-A-257 118 Marknägel bekannt, welche zur Versorgung, d.h. zur Fixation von Frakturen, bzw. Brüchen, zum Beispiel im Oberschenkel- oder Unterschenkelbereich verwendet werden. Sie umfassen einen Femurnagel und quer dazu verlaufende Knochenschrauben. Der Femurnagel ist verhältnismässig biegesteif und füllt nahezu den ganzen Markraum eines Knochens aus. Er besitzt mehrere Querbohrungen. Mit Hilfe von Knochenschrauben beidseitig der Fraktur wird der Femurnagel im Knochen fest verankert (d.h. "verriegelt"). Der Knochen ist nach der Versorgung mit einem Marknagel innerhalb kurzer Zeit wieder belastbar. Im proximalen Bereich ist der Femurnagel mit einer schrägen Durchgangsbohrung versehen, durch welche eine Schenkelhalsschraube hindurchführbar ist. Dabei ist eine Drehsicherung der Schenkelhalsschraube in der Schrägdurchbohrung vorgesehen, die eine axiale Bewegung der Schenkelhalsschraube zulässt. Diese Drehsicherung wird dadurch erreicht, dass an der Schenkelhalsschraube im Bereich der Schrägdurchbohrung am Umfang mehrere achsparallele, in Umfangsrichtung beabstandete Nuten geformt sind, in die ein in das proximale Ende des Femurnagels einsetzbarer, - sich zu diesem koaxial erstreckender - Verriegelungsbolzen eingreift. Die Nuten lassen eine Axialbewegung der Schenkelhalsschraube (quer zum Femurnagel) zu. Da der Verriegelungsstift in eine der Nuten eingreift, verhindern sie , dass sich die Schenkelhalsschraube ungewollt verdrehen kann.

Während der Knochenheilung kommt es häufig zu einer sogenannten Sinterung, wodurch sich der Knochen im Bereich der Fraktur verkürzt. Würde die Schenkelhalsschraube dieser Verkürzung nicht nachgeben, bestünde einerseits die Gefahr, dass die Schenkelhalsschraube den Femurkopf durchstösst, andererseits dass die Frakturversorgung unstabil wird.

Es sind verschiedene andere konstruktive Möglichkeiten bekannt, um die Schenkelhalsschraube in Drehrichtung zu sichern und gleichzeitig eine axiale Bewegung zuzulassen. Diese Schenkelhalsschraube ist somit gegenüber dem Femurnagel gegen Rotation gesichert, aber das proximale Knochenfragment kann sich noch immer gegenüber der Schenkelhalsschraube um deren Achse verdrehen.

Aus der US-A-2002/004590 (Basis für den Oberbegriff des Anspruchs 1) ist eine Lösung bekannt, bei der die Schenkelhalsschraube zwei einander diametral gegenüberliegend angeordnete Längsnuten aufweist, in welche ein im wesentlichen U-förmiges Verriegelungselement eingeschoben, bzw. eingetrieben wird. Diese Lösung stellt eine Kombination von Schraube und Klinge dar und vereint somit die gewünschten Eigenschaften beider Befestigungselemente. Allerdings ist die Höhe der Schenkel des Verriegelungselementes relativ gering, da sie nur in den Nuten der Schenkelhalsschraube durch den Femurnagel geführt ist und somit ist deren Wirkung als Klinge beschränkt. Durch die sich zur Schraubenspitze hin verringernde Tiefe der Längsnuten werden die Schenkel des Verriegelungselementes seitlich abgespreizt. Das Verriegelungselement dient somit nur dazu, ein Losdrehen des Knochenfragmentes von der Schenkelhalsschraube zu verhindern. Die Dreharretierung der Schenkelhalsschraube im Femurnagel erfolgt analog der EP-A-257 118 mittels eines Verriegelungsbolzens vom proximalen Ende des Femurnagels her, was für den Chirurgen beim Einsetzen und Verriegeln der Schenkelhalsschraube die Notwendigkeit bedeutet, von zwei Seiten her zu arbeiten.

Das Problem des Verdrehens wird durch aus der WO-A-01/739679 bekannte Implantatsysteme mittels zweier proximaler Verriegelungsschrauben behoben. Dabei ist jedoch ein höherer Operationsaufwand erforderlich und es besteht das Problem, dass bei kleinen Schenkelhälsen die beiden Schrauben kaum oder nicht Platz finden.

Ein anderes System verwendet eine Spiralklinge als einziges proximales Verriegelungselement, das im Nagel gegen Rotation um deren Achse gesichert ist. Das proximale Knochenfragment kann nun nicht mehr um die Achse der Klinge rotieren. In der Praxis bedeutet dies, dass die Klinge, dank der grösseren Auflagefläche, bei Belastung nicht so leicht "seitlich" durch den Knochen schneiden kann wie dies bei anderen Schrauben der Fall ist. Nachteilig ist jedoch, dass die Spiralklinge den Femurkopf u.U. nicht ausreichend in axialer Richtung stützt.

Die Spiralklinge und die Schraube gemäss US-A-2002/0045900 müssen, wie schon erwähnt, durch ein zusätzliches Element vom proximalen Ende des Femurnagels gegen Rotation gegenüber dem Femurnagel gesichert werden. Ein spezielles Problem der Schraube gemäss der US-A-2002/0045900 ist dabei die eingeschränkte Gleitfähigkeit des Gebildes Schraube/Verriegelungselement entlang der Schraubenachse in der Bohrung des Femurnagels: da die Schenkel des U-förmigen Verriegelungselements nach ihrem Durchtritt durch den Nagel von den Nuten mit geringerer Tiefe in der Schraube gespreizt werden, verklemmen sie sich u.U. Undefiniert in der Bohrung des Femurnagels.

Der Erfindung liegt somit die Aufgabe zugrunden, einen Marknagel zu schaffen, bei dem die proximale Verriegelung nur durch einen Zugang erfolgt und trotzdem das proximale Knochenfragment gut gesichert wird.

Gemäss der Erfindung wird dies dadurch erreicht, dass die Drehsicherung durch eine formschlüssige Verbindung zwischen dem Verriegelungselement und dem Femurnagel gebildet wird.

Im Falle einer Revision muss somit nur eine einzige Inzision, d.h. eine Öffnung des Patienten nur an einer Stelle von lateral am proximalen Ende der Schenkelhalsschraube erfolgen.

Damit wird erfindungsgemäss eine wirksame Drehverriegelung zwischen Marknagel und Schenkelhalsschraube erreicht, unabhängig davon, wie diese aufgebaut ist.

Die Durchgangsbohrung ist vorteilhaft mit in axialer Richtung der Schenkelhalsschraube verlaufenden Nuten versehen, die mit dem Verriegelungselement formschlüssig kooperieren. Die Schenkel des Verriegelungselementes werden somit auch in Nuten durch den Femurnagel geführt, womit eine Steigerung der Höhe der Schenkel und somit der Tragfähigkeit ermöglicht wird. Gegenüber dem bekannten U-förmigen Verriegelungselement mit beispielsweise 2mm Schenkelhöhe kann nun die Höhe wenigstens eines der Schenkel beispielsweise 3mm betragen, was einer Steigerung um 50% entspricht. Solche Nuten in der Durchgangsbohrung des Femurnagels sind beispielsweise mittels Räumwerkzeugen relativ einfach herzustellen.

Eine zweckmässige Ausführung besteht darin, dass die Nuten in der Durchgangsbohrung des Femurnagels entsprechend den Längsnuten in der Schenkelhalsschraube angeordnet sind. Dabei kann die Anzahl der Nuten in der Durchgangsbohrung mit jenen in der Schenkelhalsschraube übereinstimmen oder ungleich sein.

In einer weiteren Ausführungsform weist das Verriegelungselement mehr Schenkel auf als Nuten in der Durchgangsbohrung im Femurnagel vorgesehen sind, wobei mindestens ein Schenkel durch eine Nut in der Durchgangsbohrung des Femurnagels und mindestens ein Schenkel, - vorzugsweise derselbe - in einer Längsnut der Schenkelhalsschraube geführt ist. Somit wird die Verdrehsicherung der Schenkelhalsschraube gegenüber dem Femurnagel durch das Verriegelungselement selbst erzeugt.

Eine solche Ausführung ergibt den Vorteil, dass die Nuten im Femurnagel in Zonen geringer Beanspruchung zum proximalen und zum distalen Ende des Femurnagels hin gerichtet angeordnet werden können. Ein Schenkel in dieser Position trägt dann allerdings nur zur Rotationssicherung des proximalen Fragments bei. Der Halt in Richtung vom proximalen zum distalen Nagelende wird durch entsprechend positionierte Schenkel übernommen. Diese Schenkel liegen in Zonen, wo der Femurnagel maximal beansprucht ist, und sind deshalb bei diesem Ausführungsbeispiel nur durch Nuten in der Schenkelhalsschraube geführt.

Die Tiefe der Längsnuten in der Schenkelhalsschraube nimmt gegebenenfalls - und wie bei dem Aufbau gemäss der US-A-2002/0045900 - gegen das distale Ende der Schenkelhalsschraube hin ab. Dadurch werden die Schenkel des Verriegelungselementes beim Vortreiben radial auseinandergespreizt und eine verstärkte Dreharretierung der Schenkelhalsschraube im Knochenfragment ermöglicht.

Eine zweckmässige Ausführung besteht darin, dass die Tiefe der einzelnen Längsnuten unterschiedlich ist. Somit können die einzelnen Schenkel des Verriegelungselementes mehr oder weniger stark aufgespreizt werden und entsprechend tiefer oder weniger tief in das Knochensegment eindringen.

Die in radialer Richtung gemessene Höhe der Schenkel des Verriegelungselementes nimmt zum distalen Ende der Schenkelhalsschraube hin vorteilhaft zu. Dies ermöglicht eine gute Verankerung und somit eine besonders gute Dreharretierung des Knochenfragments. Durch eine abgestufte Höhe kann die axiale Verschiebbarkeit der Schenkelhalsschraube begrenzt werden. Die axiale Gleitfähigkeit von Schenkelhalsschraube und Verriegelungselement bleibt unbeeinträchtigt.

Eine zweckmässige Ausführung besteht darin, dass die Anzahl der Längsnuten in der Schenkelhalsschraube die Anzahl der Schenkel des Verriegelungselementes übersteigt. Das Verriegelungselement kann somit in unterschiedlicher Drehstellung axial in die Schenkelhalsschraube eingeführt und die Drehsicherung an einer oder mehreren Stellen bewirkt werden.

Die Nuten in der Schenkelhalsschraube und in der Schrägbohrung im Marknagel sind vorteilhaft gleichmässig über den Umfang verteilt angeordnet. Somit kann das Verriegelungselement in beliebiger Drehstellung in die Längsnuten der Schenkelhalsschraube eingeführt werden.

Das Verriegelungselement kann dabei nur eine, zwei, drei, vier oder eine beliebige Anzahl von Schenkeln aufweisen, sofern deren Anzahl und Lage mit den Nuten in der Schenkelhalsschraube und der Schrägbohrung kompatibel ist. Bevorzugt sind zwei Schenkel vorgesehen, die in je einer Ebene liegen, die zur anderen Ebene schräg liegt, so dass die Schenkel im Schnitt wie Teile eines V's aussehen.

Eine weitere zweckmässige Ausführung besteht darin, dass die Schenkel des Verriegelungselementes über den Umfang ungleichmässig verteilt angeordnet sind. So können beispielsweise nur zwei Schenkel, jedoch eine Vielzahl von Nuten am Femurnagel, bzw. an der Schenkelhalsschraube vorgesehen werden.

Das Verriegelungselement weist gegebenenfalls drei um ca. 120° versetzte Schenkel auf. Drei Schenkel ermöglichen eine gute Zentrierung und eine gleichmässige Kräfteverteilung über den Umfang.

Damit sich nach dem Einführen des Verriegelungselementes dieses nicht mehr verschieben kann, ist zweckmässigerweise ein Sicherungselement zur axialen Sicherung des Verriegelungselementes vorgesehen.

Eine vorteilhafte Ausführung besteht darin, dass das Sicherungselement als in das proximale Ende der Schenkelhalsschraube einschraubbarer Gewindebolzen ausgebildet ist. Das Sicherungselement kann somit bei Bedarf an der gleichen Stelle, an der auch das Verriegelungselement bedient wird, wieder herausgeschraubt und entfernt werden.

Der Femurnagel, das Verriegelungselement, die Schenkelhalsschraube und/oder das Sicherungselement bestehen zweckmässigerweise aus Titanlegierungen oder Implantatstahl.

Das dieser Erfindung zugrunde liegende Prinzip kann auch auf die Verriegelung von Nägeln oder Platten bei anderen Anwendungen im chirurgischen Bereich übertragen werden.

Die Patentansprüche sind dementsprechend breit auszulegen.

Ausbildungen der Erfindung und Varianten dazu sind in den abhängigen Patentansprüchen angegeben.

Die Bezugszeichenliste und die Zeichnung bilden, zusammen mit den in den Ansprüchen beschriebenen, beziehungsweise geschützten Gegenständen integrierende Bestandteile der Offenbarung dieser Anmeldung.

Die Figuren werden zusammenhängend und übergreifend beschrieben. Gleiche Bezugszeichen bedeuten gleiche Bauteile, Bezugszeichen mit unterschiedlichen Indices geben funktionsgleiche Bauteile an. Es zeigen dabei:
- Fig. 1: Die Einzelteile eines beispielhaften erfindungsgemässen Marknagels mit Femurnagel und Schenkelhalsschraube, in Explosionsdarstellung,
- Fig.2: den Femurnagel gemäss Fig.1 allein,
- Fig.3: eine Seitenansicht des montierten Marknagels gemäss Fig.1,
- Fig.4: eine perspektivische Darstellung des montierten Marknagels gemäss Fig.3, mit den symbolischen Umrissen des diesen umgebenden Kno- chens,
- Fig.5: eine weitere Ausführungsform, entsprechend Fig. 1,
- Fig.6: den Femurnagel gemäss Fig. 5 allein, in Ansicht,
- Fig.7: einen Ausschnitt des Marknagels gemäss Fig.5, in montiertem Zustand und
- Fig.8: eine Ansicht einer weiteren Variante des Femurnagels.

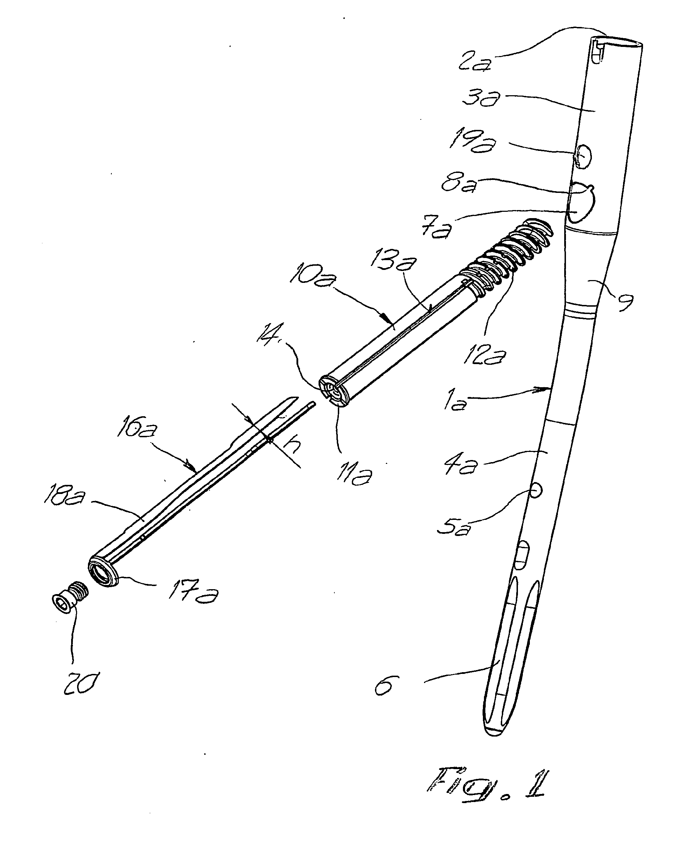

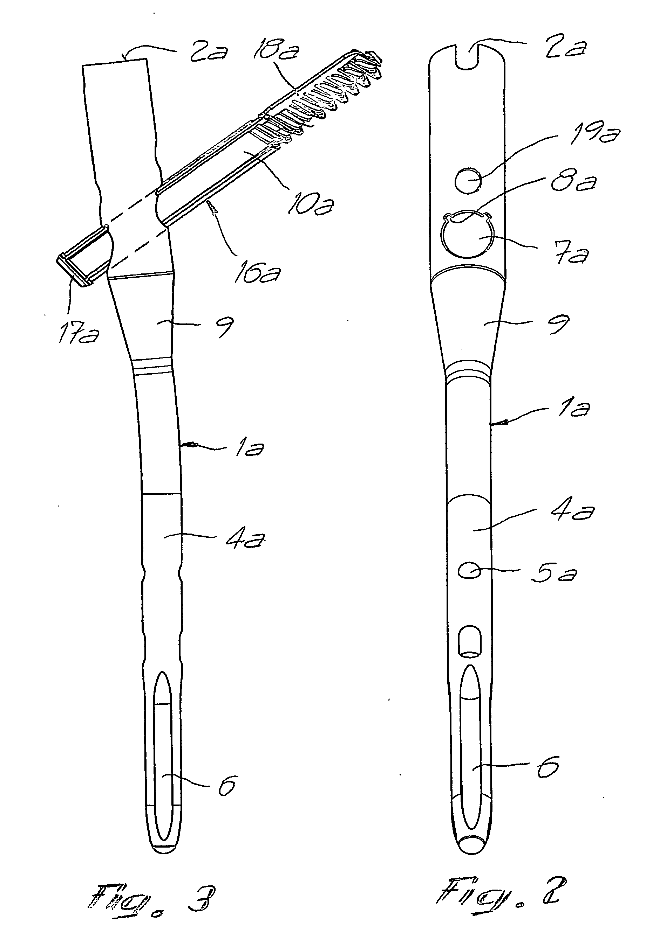

Der aus den Fig.1 bis 4 ersichtliche Marknagel besteht im wesentlichen aus einem insgesamt mit 1a bezeichneten Femurnagel und einer insgesamt mit 10a bezeichneten Schenkelhalsschraube. Der Femurnagel 1a weist ein proximales Ende 2a auf, das als Kopf 3a ausgebildet ist. Zum distalen Ende hin weist der Femurnagel 1a einen gegenüber dem Kopf 3a im Durchmesser reduzierten Schaft 4a auf. Der Übergangsbereich zwischen Kopf 3a und Schaft 4a ist als Konus 9 ausgebildet. Im vorderen Bereich ist der Schaft 4a mit wenigstens einer Querbohrung 5a versehen, welche zur axialen Sicherung des Femurnagels 1a im Knochen dient. Der Schaft 4a weist ausserdem axiale Kerben 6 auf. Der Kopf 3a ist mit wenigstens einer, schräg zur Längsachse verlaufenden, Durchgangsbohrung 7a und einer, parallel dazu verlaufenden, Hilfsbohrung 19a versehen. Durch die Hilfsbohrung 19a wird beim Eindrehen der Schenkelhalsschraube 10a ein Hilfsdraht eingeführt, um ein Mitdrehen des zu sichernden Knochenfragmentes zu verhindern.

Die Schenkelhalsschraube 10a weist an ihrem proximalen Ende einen Bund 11 auf. Der in Einführrichtung vordere, bzw. distale Bereich der Schenkelhalsschraube 10a ist mit Schraubengängen 12a versehen. Am Umfang der Schenkelhalsschraube 10a sind vier Längsnuten 13a angeordnet. Die Längsnuten 13 weisen eine sich zum distalen Ende hin verringernde Tiefe auf. Im Bereich des Bundes 11a ist eine Gewindebohrung 14 vorgesehen. Nach dem Eindrehen der Schenkelhalsschraube 10a wird ein insgesamt mit 16a bezeichnetes Verriegelungselement mit einem Kragen 17a und zwei sich in axialer Richtung erstreckenden Schenkeln 18a axial in die Längsnuten 13a der Schenkelhalsschraube 10a eingeführt. Ein Gewindebolzen 20 dient anschliessend zum Sichern des Verriegelungselementes 16a und wird in die Gewindebohrung 14 der Schenkelhalsschraube 10a eingeschraubt.

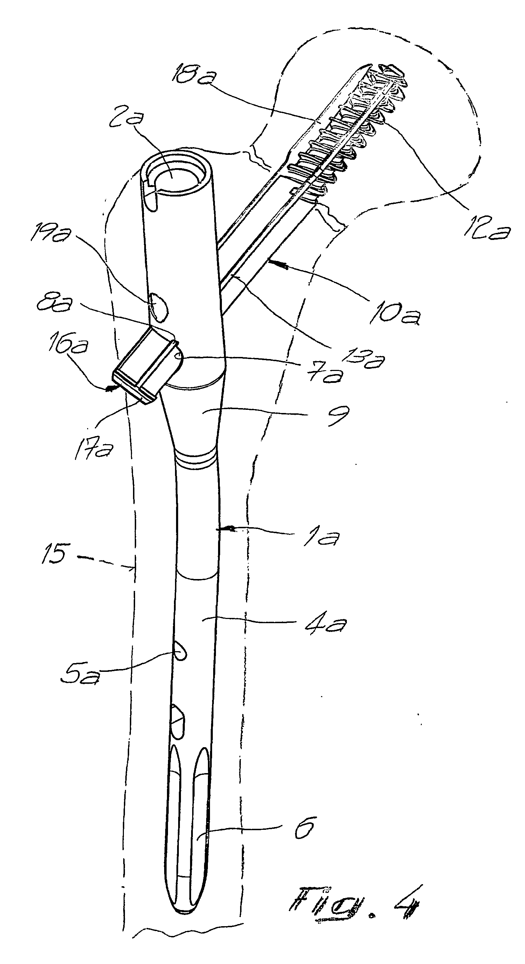

In Fig.4 sind die Umrisse des, mittels des erfindungsgemässen Marknagels zu fixierenden Knochens 15 gestrichelt dargestellt. Dabei wird besonders deutlich, dass die Schenkel 18a des Verriegelungselementes 16a, infolge der sich zum distalen Ende der Schenkelhalsschraube 10a in der Tiefe verringenden Längsnuten 13a, die Schraubengänge 12a radial überragen. Dadurch wird einerseits eine Dreharretierung der Gelenkskugel auf der Schenkelhalsschraube 10a und andererseits eine begrenzte, für die Heilung des Knochens erforderliche axiale Beweglichkeit der Schenkelhalsschraube 10a im Femurnagel 1a erreicht.

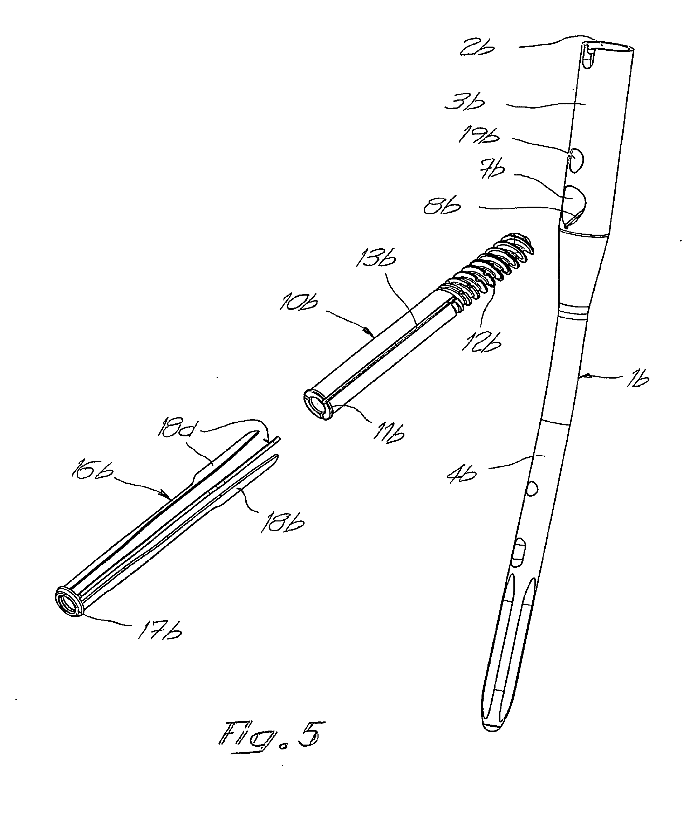

Die in Fig.5 dargestellte Ausführung unterscheidet sich von derjenigen in den Fig.1 bis 4 dadurch, dass das Verriegelungselement 16b einen Schenkel 18b und zwei Schenkel 18d aufweist und die Schenkelhalsschraube 10b mit drei gleichmäßig über den Umfang verteilt angeordneten Längsnuten 13b versehen ist. Der Femurnagel 1 b weist jedoch nur eine einzige Nut 8b zur Dreharretierung der den Femurnagel 1b durchdringenden Schenkelhalsschraube 10b auf, in die der Schenkel 18b zu liegen kommt. Die anderen beiden Schenkel 18d dienen somit lediglich der Verdrehsicherung im Knochenfragment. Diese unterschiedliche Funktion kann beispielsweise dadurch erreicht werden, dass die Schenkel 18b, 18d durch unterschiedliche Tiefe und/oder Form der Längsnuten 13b mehr oder weniger stark aufgespreizt werden. Als Variation oder in Kombination mit dieser Massnahme kann die in radialer Richtung gemessene Höhe der einzelnen Schenkel 18b, 18d auch unterschiedlich sein.

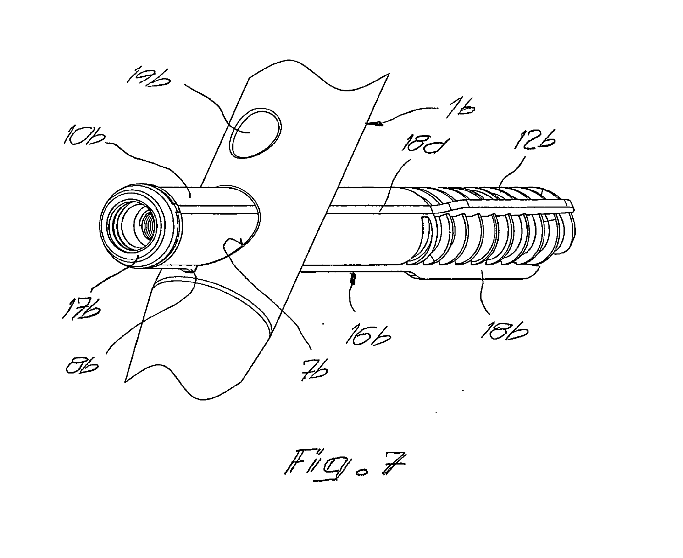

Der aus Fig.7 ersichtliche Ausschnitt zeigt die durch den Femurnagel 1 b gesteckte Schenkelhalsschraube 10b mit eingeführtem Verriegelungselement 16b. Der nach unten gerichtete Schenkel 18b ist stärker aufgespreizt als die beiden anderen Schenkel 18d. Dadurch greift der Schenkel 18b in seinem rückwärtigen Bereich formschlüssig in die Nut 8b in der Durchgangsbohrung 7b des Femumagels 1 b und bewirkt dadurch eine Dreharretierung der Schenkelhalsschraube 10b im Femurnagel 1b. Die beiden anderen Schenkel 18d überragen dagegen im aufgespreizten Zustand im Bereich des Femurnagels 1 b den Umfang der Schenkelhalsschraube 10b nicht und dienen im Bereich der Schraubengänge 12b ausschliesslich der Drehsicherung des Knochenfragments auf der Schenkelhalsschraube 10b.

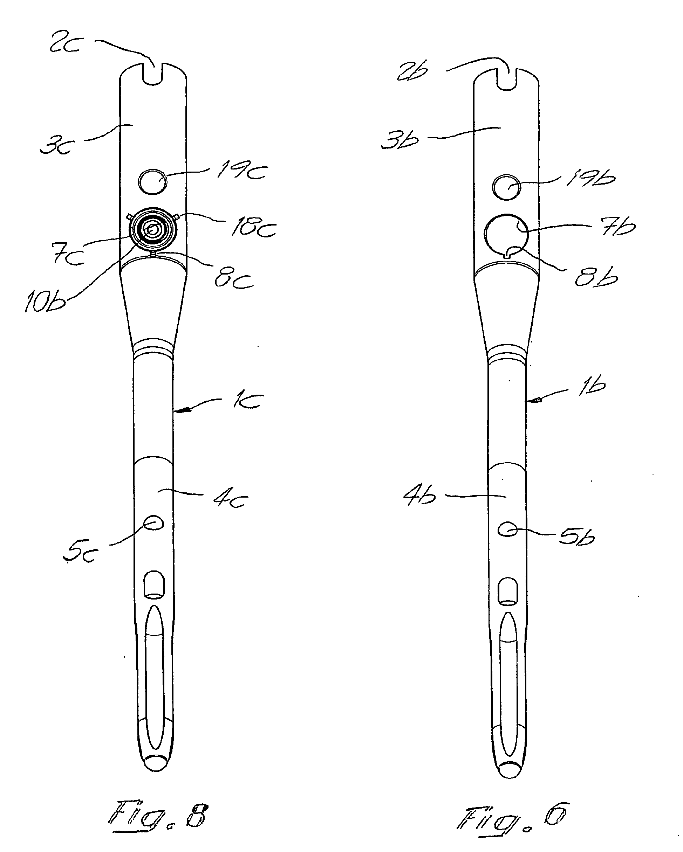

Bei der in Fig.8 dargestellten Variante sind alle drei Schenkel 18c radial gleich ausgebildet, um im eingeführten Zustand gleich weit aufgespreizt zu sein. Sie greifen formschlüssig in Nuten 8c der Durchgangsbohrung 7c im Femurnagel 1c ein. Somit übernehmen alle drei Schenkel 18c sowohl Dreharretierungsfunktion der Schenkelhalsschraube 10b im Femurnagel 1 c als auch im Knochenfragment.

### Bezugszeichenliste

- 1a, 1b, 1c: Femurnagel
- 2a, 2b, 2c: Proximales Ende
- 3a, 3b, 3c: Kopf
- 4a,4b,4c: Schaft
- 5a, 5b, 5c: Querbohrung
- 6: Kerbe
- 7a, 7b, 7c: Durchgangsbohrung
- 8a, 8b, 8c: Nut
- 9: Konus
- 10a, 10b: Schenkelhalsschraube
- 11a, 11b: Bund
- 12a, 12b: Schraubengang
- 13a, 13b: Längsnut
- 14: Gewindebohrung
- 15: Knochen
- 16a, 16b: Verriegelungselement
- 17a, 17b: Kragen
- 18a, 18b, 18c, 18d: Schenkel
- 19a, 19b, 19c: Hilfsbohrung
- 20: Gewindebolzen

## Patentansprüche

1. Marknagel zur Fixation von Frakturen des Proximalen Femur, mit einem vom proximalen Ende in den Markraum der Femur einführbaren Femurnagel (1a, 1b, 1c) und einer durch eine schräg zur Längsachse des Femurnagels verlaufende Durchgangsbohrung (7a, 7b, 7c) von lateral in den Femurnagel einführbaren Schenkelhalsschraube (10a, 10b), wobei eine die axiale Bewegung der Schenkelhalsschraube in der Durchgangsbohrung des Femurnagels ermöglichende Drehsicherung vorgesehen ist, wobei die Schenkelhalsschraube wenigstens eine in axialer Richtung verlaufende Längsnut (13a, 13b) aufweist und ein Verriegelungselement (16a, 16b) mit wenigstens einem zur Achse der Schenkelhalsschraube etwa parallel gerichteten Schenkel (18a-18b) von lateral in die Längsnut der Schenkelhalsschraube einführbar ist, **dadurch gekennzeichnet, dass** die Drehsicherung durch eine formschlüssige Verbindung zwischen dem Verriegelungselement (16a, 16b) und dem Femurnagel (1 a, 1 b, 1 c) und zwischen dem Verriegelungselement (16a, 16b) und der Schenkelhalsschraube (10a, 10b) gebildet ist.

2. Marknagel nach Anspruch 1, **dadurch gekennzeichnet, dass** wenigstens zwei axiale Längsnuten (13a, 13b) und wenigstens zwei Schenkel (18a, 18b, 18c, 18d) vorgesehen sind.

3. Marknagel nach Anspruch 1 oder 2, **dadurch gekennzeichnet, dass** die Durchgangsbohrung (7a, 7b, 7c) mit in axialer Richtung verlaufenden Nuten (8a, 8b, 8c) versehen ist, die mit dem Verriegelungselement (16a, 16b) formschlüssig kooperieren.

4. Marknagel nach Anspruch 3, **dadurch gekennzeichnet, dass** die Nuten in der Durchgangsbohrung (7a, 7b, 7c) des Femurnagels (1a, 1 b, 1 c) in Anzahl und Relativlage entsprechend den Längsnuten (13a, 13b) in der Schenkelhalsschraube (10a, 10b) angeordnet sind.

5. Marknagel nach einem der vorhergehenden Ansprüche, **dadurch gekennzeichnet, dass** das Verriegelungselement (16b) mehr Schenkel (18b, 18d) als Nuten (8b) in der Durchgangsbohrung (7b) des Femurnagels (1 b) aufweist wobei mindestens ein Schenkel (18b) durch eine Nut (8b) in der Durchgangsbohrung (7b) des Femurnagels (1 b) und mindestens ein Schenkel (18d), - vorzugsweise derselbe - in einer Längsnut (13b) der Schenkelhalsschraube (10b) geführt ist.

6. Marknagel nach einem der vorhergehenden Ansprüche, **dadurch gekennzeichnet, dass** die Tiefe der Längsnuten (13a, 13b) in der Schenkelhalsschraube (10a,10b) gegen das distale Ende hin abnimmt.

7. Marknagel nach einem der vorhergehenden Ansprüche, **dadurch gekennzeichnet, dass** die Tiefe der einzelnen Längsnuten (13a, 13b) unterschiedlich ist.

8. Marknagel nach einem der vorhergehenden Ansprüche, **dadurch gekennzeichnet, dass** die in radialer Richtung gemessene Höhe (h) der Schenkel (18a, 18b, 18c, 18d) des Verriegelungselementes (16a, 16b) zum distalen Ende hin - gegebenenfalls stufenförmig - zunimmt.

9. Marknagel nach einem der vorhergehenden Ansprüche, **dadurch gekennzeichnet, dass** die Anzahl der Längsnuten (13a) die Anzahl der Schenkel (18a) des Verriegelungselementes (16a) übersteigt.

10. Marknagel nach einem der vorhergehenden Ansprüche, **dadurch gekennzeichnet, dass** die Schenkel (18a, 18b, 18c, 18d) des Verriegelungselementes (16a, 16b) über den Umfang gleichmässig verteilt angeordnet sind.

11. Marknagel nach einem der vorhergehenden Ansprüche, **dadurch gekennzeichnet, dass** die Schenkel (18a) des Verriegelungselementes (16a) über den Umfang ungleichmässig verteilt angeordnet sind, wobei die Schenkel (18a) im Schnitt vorzugsweise wie Teile eines V's, X's oder Y's aussehen.

12. Marknagel nach einem der vorhergehenden Ansprüche, **dadurch gekennzeichnet, dass** das Verriegelungselement (16b) drei Schenkel (18c) aufweist.

13. Marknagel nach einem der vorhergehenden Ansprüche, **dadurch gekennzeichnet, dass** die Schenkel (18a, 18b) des Verriegelungselements (16a, 16b) an ihrem proximalen Ende durch einen scheiben- oder ringförmigen Kragen (17a, 17b) verbunden sind.

14. Marknagel nach einem der vorhergehenden Ansprüche, **dadurch gekennzeichnet, dass** ein Sicherungselement zur axialen Sicherung des Verriegelungselementes vorgesehen ist.

15. Marknagel nach Anspruch 11, **dadurch gekennzeichnet, dass** das Sicherungselement als durch den Kragen (17a, 17b) in das proximale Ende der Schenkelhalsschraube (10a, 10b) einschraubbarer Gewindebolzen (20) ausgebildet ist.

16. Marknagel nach einem der vorhergehenden Ansprüche, **dadurch gekennzeichnet, dass** der Femurnagel (1a, 1 b, 1c), die Schenkelhalsschraube (10a, 10b), das Verriegelungselement (16a, 16b) und/oder das Sicherungselement (20) aus Titanlegierungen oder Implantatstahl bestehen.

## Claims

1. A medullary nail for fixation of fractures of the proximal femur, having a femur nail (1a, 1b, 1c) insertable into the medullary space of the femur from the proximal end and having a femur neck screw (10a, 10b) insertable through a through-bore (7a, 7b, 7c) running obliquely to the longitudinal axis of the femur nail, such that the screw is insertable laterally into the femur nail, wherein a locking mechanism, which allows axial movement of the femur neck screw in the through-bore of the femur nail, is provided, wherein the femur neck screw has at least one longitudinal groove (13a, 13b) running in the axial direction and a locking element (16a, 16b) having at least one leg (18a-18d) directed approximately parallel to the axis of the femur neck screw being insertable laterally into the longitudinal groove of the screw, **characterized in that** the locking mechanism is formed by a form-fitting connection between the locking element (16a, 16b) and the femur nail (1a, 1b, 1c) and between the locking element (16a, 16b) and the screw (10a, 10b).

2. The medullary nail according to Claim 1, **characterized in that** at least two axial longitudinal grooves (13a, 13b) and at least two legs (18a, 18b, 18c, 18d) are provided.

3. The medullary nail according to Claim 1 or 2, **characterized in that** the through-bore (7a, 7b, 7c) is provided with grooves (8a, 8b, 8c) running in the axial direction and cooperating with the locking element (16a, 16b) in a form-fitting manner.

4. The medullary nail according to Claim 3, **characterized in that** grooves are arranged in the through-bore (7a, 7b, 7c) of the femur nail (1a, 1b, 1c), corresponding in number and relative position to the longitudinal grooves (13a, 13b) in the screw (10a, 10b).

5. The medullary nail according to any one of the preceding claims, **characterized in that** the locking element (16b) has more legs (18b, 18d) than grooves (8b) in the through-bore (7b) of the femur nail (1b), wherein at least one leg (18b) is guided through a groove (8b) in the through-bore (7b) of the femur nail (1b) and at least one leg (18d), preferably the same leg, is guided in a longitudinal groove (13b) of the screw (10b).

6. The medullary nail according to any one of the preceding claims, **characterized in that** the depth of the longitudinal grooves (13a, 13b) in the screw (10a, 10b) decreases toward the distal end.

7. The medullary nail according two any one of the preceding claims, **characterized in that** the depth of the individual longitudinal grooves (13a, 13b) varies.

8. The medullary nail according to any one of the preceding claims, **characterized in that** the height (h) of the legs (18a, 18b, 18c, 18d) of the locking element (16a, 16b), as measured in the radial direction, increases toward the distal end, optionally in steps.

9. The medullary nail according to any one of the preceding claims, **characterized in that** the number
of longitudinal grooves (13a) exceeds the number of legs (18a) of the locking element (16a).

10. The medullary nail according to any one of the preceding claims, **characterized in that** the legs (18a, 18b, 18c, 18d) of the locking element (16a, 16b) are arranged so they are distributed uniformly over the circumference.

11. The medullary nail according to any one of the preceding claims, **characterized in that** the legs (18a) of the locking element (16a) are arranged so that they are unevenly distributed over the circumference, such that the legs (18a) appear like parts of a V, X or Y shape, preferably in cross section.

12. The medullary nail according to any one of the preceding claims, **characterised in that** the locking element (16b) has three legs (18c).

13. The medullary nail according to any one of the preceding claims, **characterized in that** the legs (18a, 18b) of the locking element (16a, 16b) are connected at their proximal ends by a disc-shaped or ring-shaped collar (17a, 17b).

14. The medullary nail according to any one of the preceding claims, **characterized in that** a securing element is provided for securing the locking element axially.

15. The medullary nail according to Claim 11, **characterized in that** the securing element is designed as a threaded bolt (20), which can be screwed through the collar (17a, 17b) into the proximal end of the screw (10a, 10b).

16. The medullary nail according to any one of the preceding claims, **characterized in that** the femur nail (1a, 1b, 1e), the screw (10a, 10b), the locking element (16a, 16b) and/or the securing element (20) are made of titanium alloys or implant-grade steel.

## Revendications

1. Broche pour la fixation de fractures du fémur proximal, comportant une broche fémorale (1a, 1b, 1c) pouvant être introduite depuis l'extrémité proximale dans l'espace moelleux du fémur et une vis pour col du fémur (10a, 10b) pouvant être introduite latéralement dans la broche fémorale par un alésage traversant (7a, 7b, 7c) s'étendant obliquement par rapport à l'axe longitudinal de la broche fémorale, étant prévu une sécurité anti-rotation permettant le mouvement axial de la vis pour col du fémur dans l'alésage traversant, la vis pour col du fémur présentant au moins une rainure longitudinale (13a, 13b) s'étendant dans le sens axial et un élément de verrouillage (16a, 16b) comportant au moins une branche (18a-18d) orientée à peu près parallèlement à l'axe de la vis pour col du fémur pouvant être introduit latéralement dans la rainure longitudinale de la vis pour col du fémur, **caractérisée en ce que** la sécurité anti-rotation est constituée par une liaison en correspondance géométrique entre l'élément de verrouillage (16a, 16b) et la broche fémorale (1a, 1b, 1c) et entre l'élément de verrouillage (16a, 16b) et la vis pour col du fémur (10a, 10b).

2. Broche selon la revendication 1, **caractérisée en ce qu'**il est prévu au moins au moins deux rainures longitudinales axiales (13a, 13b) et au moins deux branches (18a, 18b, 18c, 18d).

3. Broche selon la revendication 1 ou 2, **caractérisée en ce que** l'alésage traversant (7a, 7b, 7c) est pourvu de rainures (8a, 8b, 8c) s'étendant dans le sens axial et coopérant en correspondance géométrique avec l'élément de verrouillage (16a, 16b).

4. Broche selon la revendication 3, **caractérisée en ce que** les rainures sont disposées dans l'alésage traversant (7a, 7b, 7c) de la broche fémorale (1a, 1b, 1c) en nombre et en position relative correspondant aux rainures longitudinales (13a, 13b) de la vis pour col du fémur (10a, 10b).

5. Broche selon une des revendications précédentes, **caractérisée en ce que** l'élément de verrouillage (16b) présente plus de branches (18b, 18d) que de rainures (8b) dans l'alésage traversant (7b) de la broche fémorale (1b), au moins une branche (18b) étant guidée par une rainure (8b) dans l'alésage traversant (7b) de la broche fémorale (1b) et au moins une branche (18d) - de préférence la même - dans une rainure longitudinale (13b) de la vis pour col du fémur (10b).

6. Broche selon une des revendications précédentes, **caractérisée en ce que** la profondeur des rainures longitudinales (13a, 13b) de la vis pour col du fémur (10a, 10b) diminue en direction de l'extrémité distale.

7. Broche selon une des revendications précédentes, **caractérisée en ce que** la profondeur des rainures longitudinales distinctes (13a, 13b) est variable.

8. Broche selon une des revendications précédentes, **caractérisée en ce que** la hauteur (h) mesurée dans le sens radial des branches (18a, 18b, 18c, 18d) de l'élément de verrouillage (16a, 16b) augmente en direction de l'extrémité distale - le cas échéant graduellement.

9. Broche selon une des revendications précédentes, **caractérisée en ce que** le nombre de rainures longitudinales (13a) dépasse le nombre de branches (18a) de l'élément de verrouillage (16a).

10. Broche selon une des revendications précédentes, **caractérisé en ce que** les branches (18a, 18b, 18c, 18d) de l'élément de verrouillage (16a, 16b) sont réparties régulièrement sur la circonférence.

11. Broche selon une des revendications précédentes, **caractérisée en ce que** les branches (18a) de l'élément de verrouillage (16a) sont réparties irrégulièrement sur la circonférence, les branches (18a) ayant en coupe de préférence l'aspect d'un V, d'un X ou d'un Y.

12. Broche selon une des revendications précédentes, **caractérisée en ce que** l'élément de verrouillage (16b) présente trois branches (18c).

13. Broche selon une des revendications précédentes, **caractérisée en ce que** les branches (18a, 18b) de l'élément de verrouillage (16a, 16b) sont reliées par leur extrémité proximale par une collerette en forme de disque ou d'anneau (17a, 17b).

14. Broche selon une des revendications précédentes, **caractérisée en ce qu'**il est prévu un élément de blocage pour le blocage axial de l'élément de verrouillage.

15. Broche selon la revendication 11, **caractérisée en ce que** l'élément de blocage se présente sous la forme d'un boulon fileté (20) pouvant être vissé à travers la collerette (17a, 17b) dans l'extrémité proximale de la vis pour col du fémur (10a, 10b).

16. Broche selon une des revendications précédentes, **caractérisée en ce que** la broche fémorale (1a, 1b, 1c), la vis pour col du fémur (10a, 10b), l'élément de verrouillage (16a, 16b) et/ou l'élément de blocage (20) sont composés d'alliages de titane ou d'acier pour implantes.
